# EUROPEAN PATENT SPECIFICATION

(11) **EP 1 563 301 B1**
(45) Date of publication and mention of the grant of the patent: **14.09.2011**
(21) Application number: 03789967.1
(22) Date of filing: 20.11.2003
(51) Int. Cl.: G01N 33/536

(54) **VACCINE FOR THE PREVENTION OF MALARIA**
IMPFSTOFF ZUR VORBEUGUNG GEGEN MALARIA
VACCINE DE PREVENTION DU PALUDISME

(30) Priority: 20.11.2002 US 427911 P
(43) Date of publication of application: 17.08.2005
(73) Proprietor: Sanaria Inc., Rockville, MD 20850 (US)
(72) Inventor: HOFFMAN, Stephen, L, Gaithersburg, MD 20878 (US); LUKE, Thomas, C, Brookville, MD 20833 (US)
(74) Representative: Wibbelmann, Jobst
(86) International application number: PCT/US2003/037498
(87) International publication number: WO 2004/045559

(56) References cited:
- WO-A2-2009/090269
- US-A- 5 766 597
- HOFFMAN STEPHEN L ET AL: "Protection of humans against malaria by immunization with radiation-attenuated Plasmodium falciparum sporozoites" JOURNAL OF INFECTIOUS DISEASES, vol. 185, no. 8, 15 April 2002 (2002-04-15), pages 1155-1164, XP002435268 ISSN: 0022-1899
- CLYDE D F: "IMMUNITY TO FALCIPARUM AND VIVAX MALARIA INDUCED BY IRRADIATED SPOROZOITES A REVIEW OF THE UNIVERSITY OF MARYLAND MARYLAND USA STUDIES 1971-75" BULLETIN OF THE WORLD HEALTH ORGANIZATION. BULLETIN DE L'ORGANISATION MONDIALE DE AL SANTE, GENEVA, CH, vol. 68, no. SUPPL, 1990, pages 9-12, XP009084214 ISSN: 0366-4996
- JIANG J-B ET AL: "Induction of retarded exoerythrocytic schizonts by chloroquanide resulting in delayed parasitaemia of Plasmodium inui in Macaca mulatta" ACTA PHARMACOLOGICA SINICA 1990 CHINA, vol. 11, no. 3, 1990, pages 272-274, XP002435270 ISSN: 0253-9756
- COLLINS WILLIAM E ET AL: "Adaptation of a strain of Plasmodium vivax from Mauritania to New World monkeys and anopheline mosquitoes" JOURNAL OF PARASITOLOGY, vol. 84, no. 3, June 1998 (1998-06), pages 619-621, XP002435271 ISSN: 0022-3395
- KRZYCH V ET AL: "T Lymphocytes from Volunteers Immunized with Irradiated Plasmodium falciparum Sporozoites Recognized Liver and Blood Stage Malaria Antigens" JOURNAL OF IMMUNOLOGY, THE WILLIAMS AND WILKINS CO. BALTIMORE, US, vol. 155, 1995, pages 4072-4077, XP002984522 ISSN: 0022-1767
- NUSSENZWEIG R: "Use of radiation-attenuated sporozoites in the immunoprophylaxis of malaria." INTERNATIONAL JOURNAL OF NUCLEAR MEDICINE AND BIOLOGY 1980, vol. 7, no. 2, 1980, pages 89-96, XP002435272 ISSN: 0047-0740
- KRAMER L D ET AL: "INTRA MUSCULAR IMMUNIZATION OF MICE WITH IRRADIATED PLASMODIUM-BERGHEI SPOROZOITES ENHANCEMENT OF PROTECTION WITH ALBUMIN" AMERICAN JOURNAL OF TROPICAL MEDICINE AND HYGIENE, vol. 24, no. (6 PART 1), 1975, pages 913-916, XP009084197 ISSN: 0002-9637
- COLLINS, W. E. ET AL.: 'Potential of the Panama Strain of Plasmodium vivax for the Testing of Malarial Vaccines in Aotus Nancymai Monkeys' AMERICAN JOURNAL OF TROPICAL MEDICINE AND HYGIENE vol. 67, no. 5, 2002, pages 454 - 458, XP002976724
- ZAPATA, J. C. ET AL.: 'Reproducible infection of intact aotus lemurinus griseimembra monkeys by plasmodium falciparum sporozoite inoculation' JOURNAL OF PARASITOLOGY vol. 88, no. 4, August 2002, pages 723 - 729, XP002976725
- SCHOFIELD ET AL.: 'Synthetic GPI as a candidate anti-toxic vaccine in a model of malaria' NATURE vol. 418, August 2002, pages 785 - 789, XP002239679

## Description

### CROSS REFERENCE TO RELATED APPLICATIONS

### FIELD OF THE INVENTION

This application relates to preventing malaria by administering a vaccine. More particularly, this invention relates to a vaccine against malaria infection comprising the administration of attenuated sporozoites to a human or animal.

### INTRODUCTION AND DESCRIPTION OF THE PRIOR ART

Malaria is a disease that affects 300-500 million people, kills one to three million individuals annually, and has an enormous economic impact on people in the developing world, especially those in sub Saharan Africa [1, 2]. *Plasmodium falciparum* accounts for the majority of deaths from malaria in the world. The World Tourist Organization reported that of the nearly 700 million international tourist arrivals recorded worldwide in 2000, approximately 9 million were to West, Central or East Africa, 37 million were to South-East Asia, 6 million to South Asia and 10 million to Oceania [3]. It is estimated that more than 10,000 travelers from North America, Europe, and Japan contract malaria per year. For more than 100 years during every military campaign conducted where malaria was transmitted, the U.S. forces have had more casualties from malaria than from hostile fire. An estimated 12,000,000 person days were lost during World War II and 1.2 million during the Vietnam conflict due to malaria [4].

Transmission of the parasite *Plasmodium* (the protozoan parasite causing malaria) occurs via the bite of infected female *Anopheles* mosquitoes, which are active from dusk to dawn. Sporozoites migrate from the bite site to the liver via the blood stream, where they multiply within hepatocytes, producing, in the case of *P. falciparum,* 10,000-40,000 progeny per infected cell. These liver stage parasites express a set of antigens which are not expressed in sporozoites. This new generation of parasites re-enters the blood stream as merozoites, expressing a set of antigens which are different from those expressed during the sporozoite and early hepatic stages, and invade erythrocytes, where additional multiplication increases parasite numbers by approximately 10 to 20 fold every 48 hours. Unlike the five to ten day development in the liver, which does not induce any symptoms or signs of illness, untreated blood stage infection causes hemolysis, shaking chills, high fevers, and prostration. In the case of *P*. *falciparum,* the most dangerous of the four species of *Plasmodium* that infect humans, the disease is complicated by disruption of microcirculatory blood flow and metabolic changes in vital organs such as the brain, kidney and lung, frequently leading to death if not urgently treated.

An effective vaccine against *P. falciparum* malaria remains one of the great challenges of medicine. Despite over one hundred years of effort, hundreds of millions of dollars in research, lifelong sacrifice from dedicated physicians and scientists, and many promising experimental vaccines, there is no marketed vaccine to alleviate one of the great infectious scourges of humanity. A generation ago, public health initiatives employing chloroquine, DDT and vector control programs seemed poised to consign *falciparum* malaria to insignificance as a worldwide menace. The lack of an effective vaccine complicated these efforts, but sustainable control seemed imminent.

The promise of impending success was short lived and the reasons for failure were multi-factorial. The parasites grew increasingly resistant to highly effective and affordable anti-malarial medications, vector control measures lapsed, and trans-migration, war and economic disruption became increasingly more common in endemic areas of the developing world. As a result, *falciparum* malaria has resurged, annually placing 2.5 billion humans at risk, causing 300-900 million infections, and killing 1- 3 million people. Of the many social, economic, environmental and political problems that afflict the developing world, *P. falciparum* malaria is increasingly seen as both a root cause and cruel result of these inequities, and is a singular impediment to solving these complex problems. Controlling *falciparum* malaria in the developing world may be possible without an effective vaccine. In practice, given social, political and economic realities, the inventors believe that a vaccine may be an essential component of a sustainable control program, and will be required for a global eradication campaign.

It is in this context that the modern period of malaria vaccine development has been particularly frustrating. Since the early 1980's, breathtaking technological advances in molecular biology and medical science have occurred. These advances accelerated the identification of stage-specific *P. falciparum* proteins and epitopes, and host immune mechanisms and responses. This knowledge was translated into a range of novel vaccine candidates [5,6]. In one sense, this modem period has been the golden age of malaria vaccine research and human testing. However, in spite of the Herculean efforts of malaria researchers, the majority of these vaccines have failed to provide any protective immunity in humans - with only one demonstrating reproducible short term protection against infection in 40%-70% of recipients [7-9].

Given enough time and resources, these vaccine strategies, or others yet to be developed, may ultimately lead to a robust vaccine. However, at a recent Keystone meeting, "Malaria's Challenge: From Infants to Genomics to Vaccines" [6], the attendees were polled as to when they thought a malaria vaccine might be "launched" as a commercial product. Many in the room indicated that they thought the first vaccine would not be launched until 2016-2025. The leader of Glaxo Smith Kline's (GSK) efforts to develop a recombinant *P. falciparum* circumsporozoite protein (PfCSP) vaccine voiced the most optimism. It was indicated that if all went well, this single protein vaccine could be "launched" in 7-8 years (2009-2010). Given that GSK and the U.S. Army have been working on a recombinant protein PfCSP vaccine since the 1984 cloning of the PfCSP [10], and that many malariologists express concern as to whether a single protein vaccine will be adequate to sustainably control malaria, this time line of more than 25 years for development of a single protein vaccine places a chillingly realistic perspective, on the possibilities for developing vaccines that will truly reduce the burden of this disease.

**Protective Immunity After Immunization with Radiation Attenuated Sporozoites:** In 1967 Nussenzweig reported that intravenous administration of radiation attenuated *P. berghei* sporozoites to A/J mice protected the mice against challenge with infectious *P. berghei* sporozoites [11]. These rodent studies provided the impetus for human studies, and by the early 1970s, two groups established that immunizing human volunteers with the bites of irradiated mosquitoes carrying *P. falciparum* sporozoites in their salivary glands could protect volunteers against challenge with fully infectious *P. falciparum* sporozoites [12-19]. These studies demonstrated that a malaria vaccine offering sterile protective immunity was possible. However, the only way to produce sporozoites at that time was to infect a volunteer with *P. falciparum,* treat the volunteer with doses of chloroquine to suppress but not eliminate the parasite, allow gametocytes to develop, and then feed mosquitoes on these volunteers. Even if one could produce sporozoites in adequate numbers by this method, it was considered clinically, technically and logistically impractical to immunize humans with an irradiated sporozoite vaccine. In large part this was because the sporozoites had to be delivered alive, either by the bite of infected mosquitoes, or by intravenous injection as was done with mice. The scientists active in the field concluded that other routes of immunization would not provide adequate or comparable protection as compared to immunization by intravenous injection or by the bite of infected mosquitoes; in essence ruling out the use of attenuated sporozoites as a vaccine from their perspective. The published views of several such scientists are quoted below.

"This observation corroborates previous reports (Nussenzweig, Vanderberg and Most, 1967 and 1969) and extends their findings. Groups of mice immunized by other parenteral routes (i.m., i.p., and i.c) exhibited an overall level of protection much lower than the i.v. immunized mice." [20]

"These studies have confirmed a previous report which demonstrated that intramuscularly injected irradiated sporozoites of *P. berghei* are far less effective than those injected intravenously in protectively immunizing mice against sporozoite-induced malaria...The chief limitation preventing an extension to human trials was the requirement for intravenous immunization a procedure posing unacceptable medical risks." (In the study referred to in this quotation, protection by the intramuscular route ranged between 11 % and 42% and protection by the subcutaneous route was 0%) [21].

"It was further shown that of the various routes of immunization used in vaccination attempts in rodents (i.m., i.v., subcutaneous, per os, etc.) the intravenous route gave the highest degree of protection and most reproducible results. The only other very effective route of immunization is by the bite of infected, irradiated mosquitoes." [22]. In this 1980 review, "Use of Radiation-attenuated Sporozoites in the Immunoprophylaxis of Malaria," Dr. Nussenzweig goes on to discuss the potential for developing a sporozoite malaria vaccine, and concludes, "In conclusion, recent findings appear to indicate that we now have the necessary powerful tools which should provide the means to clarify the mechanism of sporozoite-induced immunity and to isolate the protective antigens. Under these conditions, the various obstacles to the development of a sporozoite vaccine for malaria appear to be surmountable, hopefully in the not too remote future." Dr. Nussenzweig does not discuss the idea of utilizing a whole attenuated sporozoite vaccine as a reasonable alternative, only the use of sporozoites to provide the components of a vaccine that induces immunity against the sporozoite stage.

In 1980 after nearly 15 years of work on the irradiated sporozoite vaccine model, it was concluded by the unquestioned leader in the field, Dr. Nussenzweig, that the route to a vaccine lay through modem science; understanding immunologic mechanisms of protection and the antigenic targets of those protective immune responses, and constructing a "subunit" sporozoite vaccine. From then onwards there was essentially no mention or discussion in the literature of trying to develop an attenuated whole parasite sporozoite vaccine as a practical vaccine for humans for many reasons, not the least of which was that despite these 15 years of research, no scientists had discovered a reasonable approach to administering sporozoites other than by intravenous administration or by the bite of infected mosquitoes.

There was also no further work to develop an attenuated sporozoite vaccine, because the sporozoites would have to be raised in aseptic mosquitoes, aseptically purified, and suitably preserved and reconstituted prior to administration, and after such treatment would still have to be able to elicit protective immune responses when administered.

Potential solutions to parts of the problems of production, though not recognized at the time as being related to developing an attenuated sporozoite vaccine, were being reported. In 1975, a method for culturing *P. falciparum* in vitro was reported [23, 24], followed in 1982 by a method for producing gametocytes from these cultures [24]. In 1986, it was reported that humans could be infected by the sporozoites produced in mosquitoes that had fed on these in vitro cultures [26]. There was therefore a way to produce sporozoites without the difficulties of *in vivo* production of gametocytes in humans. These developments on their own were not adequate to overcome all of the obstacles to development of attenuated sporozoite vaccine. There was not a way to produce enough of the sporozoites or produce and process the sporozoites under conditions that met regulatory standards. Furthermore, there were no data indicating that properly produced and processed sporozoites could be administered successfully in a clinically acceptable and practical manner.

Thus, following the failure of the malaria scientific community to discover a method to deliver attenuated sporozoites in a clinically acceptable and practical manner sufficient to achieve high level protection, the attenuated sporozoite vaccine was dropped from clinical consideration, and the community as presaged by Dr. Nussenzweig (paragraph [012] above) embraced modem molecular science in the hope of developing a vaccine. Several promising developments launched the modern era of malaria sub-unit vaccine development. A monoclonal antibody against the major surface protein of sporozoites, the circumsporozoite protein (CSP), had been produced and shown to protect mice in passive transfer experiments [27]. Additionally, the gene encoding the PfCSP protein had been cloned and sequenced [10]. Coincidentally, the first purified recombinant protein vaccine, the hepatitis B surface antigen vaccine, was developed and marketed [28]. The weight of evidence and trends in vaccine science seemed to offer malaria researchers a roadmap to quickly develop a human malaria vaccine. Since it was considered impractical to produce and administer the sporozoite vaccine, returning to an attenuated whole parasite vaccine seemed unnecessary and dated, and all subsequent efforts focused on the promise of sub-unit vaccines.

In 1987 when the first recombinant protein [29] and synthetic peptide [30] vaccines did not prove to be as protective as expected, instead of considering the development of an attenuated sporozoite vaccine which was considered impossible to produce and administer, scientists focused on understanding the immune mechanisms responsible for protective immunity, and the antigenic targets of these protective immune responses, and developing subunit vaccines and vaccine delivery systems that induced such protection. Much of this basic work was carried out in the *P. berghei* and *P. yoelii* rodent model systems. This rodent malaria work provided important insights into immunologic mechanisms and antigenic targets of irradiated sporozoite vaccine-induced protection and led to the development of a number of candidate vaccines [31-33]. None of these studies which were conducted after the cloning of the gene encoding the *P. falciparum* circumsporzoite protein (PfCSP) in 1984 through the end of the millenium suggested the possibility of developing a human irradiated whole sporozoite vaccine, because none of the investigators thought it was possible to produce or administer such a vaccine in a practical manner. Interestingly, sub-unit (recombinant protein, synthetic peptide, recombinant virus, DNA plasmid) vaccine formulations have been shown to produce excellent protection in mice, but nothing comparable in humans. In contrast the protection in mice by intravenous administration of irradiated sporozoites [11] led to human studies, that demonstrated that exposure to the bites of irradiated mosquitoes with *P. falciparum* sporozoites in their salivary glands induced protection [34].

In 1989, after a number of disappointing clinical trials of sub-unit PfCSP vaccines, immunization of volunteers by the bites of mosquitoes carrying *P. falciparum* sporozoites in their salivary glands and then attenuated by exposure in vivo to gamma radiation was begun at the Naval Medical Research Institute later Naval Medical Research Center (NMRI later NMRC) and Walter Reed Army Institute of Research (WRAIR). The goal of this research was to better delineate the clinical characteristics and requirements that led to protecting humans with the irradiated sporozoite vaccine, assess the protective immune responses elicited in humans, and identify the antigens and epitopes on those proteins that elicited immune responses in humans. It was never a consideration to develop irradiated sporozoites as a human vaccine, as it was considered completely impractical and technically unfeasible to produce such a vaccine as well as to administer such a vaccine. Preliminary clinical results and extensive immunological assay results from these studies were published [35-41]. These immunological studies combined with those of others on this subject [42-48] increased our understanding of the immunological responses in humans immunized with radiation attenuated *P. falciparum* sporozoites. However, there was no consideration or mention of trying to develop an attenuated sporozoite vaccine.

The results of the first 10 years' clinical experience with these immunizations and challenges were recently reported, and combined with all the published clinical reports of immunizing humans with irradiated *Plasmodium* sporozoites [34] from the University of Maryland (1970's, late 1980's and early 1990's), and the Rush - Presbyterian - St Luke's Medical Centre in Chicago and the Naval Medical Research Institute in the 1970's [12-19, 34]. A number of observations arose from the analysis that was conducted.

A). There was a dose response in regard to protective immunity among volunteers challenged by the bite of 5-14 infected mosquitoes. Thirteen of 14 volunteers (93%) immunized by the bites of greater than 1000 infected, irradiated mosquitoes were protected against developing blood stage *P. falciparum* infection when challenged within 10 weeks of their last primary immunization. There were 35 challenges of these volunteers and there was complete protection against development of blood stage infection in 33 of the 35 challenges (94%). Four of 10 volunteers (40%) immunized by the bite of greater than 200 and less than 1000 infected, irradiated mosquitoes were protected against developing blood stage *P. falciparum* infection when challenged within 10 weeks of their last primary immunization, a significantly lower level of protective immunity than among volunteers immunized with > 1000 infective bites (p= 0.0088, Fisher's exact test, 2-tailed). There were 15 challenges of the volunteers immunized with less than 1000 infective bites, and there was complete protection against development of blood stage infection in 5 of the 15 challenges (33%), a significantly lower level of protective immunity than among volunteers immunized with > 1000 infective bites (p= 0.000015, Fisher's exact test, 2-tailed).

B). Protective immunity lasted for at least 42 weeks (10.5 months). Five of 6 of the above 14 volunteers when challenged from 23 to 42 weeks (23, 36, 39,41, and 42 weeks) after their last primary or secondary immunization were protected against experimental challenge. Except for a single challenge of one volunteer five years after last immunization (not protected), there were no other challenges assessing longevity of protective immunity.

C). Protection was not strain specific. Four volunteers were challenged with isolates of *P. falciparum* different than the isolates with which they were immunized. The four volunteers were completely protected in seven of seven such challenges with different isolates of *P. falciparum.*

D). Immunologic memory lasts for at least 5 years. A volunteer who had been exposed to the bite of 1601 irradiated infected mosquitoes, and protected when challenged 9 and 42 weeks after last exposure, was not protected when re-challenged 5 years after last exposure to to irradiated, infected mosquitoes. He was treated for his malaria, boosted by exposure to 147 irradiated, infected mosquitoes, and re-challenged by exposure to the bite of 5 non-irradiated mosquitoes infected with *P. falciparum* sporozoites. This volunteer was protected against that infectious challenge [34], demonstrating that the protective immunity was boostable with a single exposure to irradiated sporozoites.

Thus, protection was achieved in greater than 90% of challenge experiments after greater than 1000 mosquito bites, lasted for at least 10.5 months, and was not *P. falciparum* isolate (strain) specific.
A "sub-unit" vaccine demonstrating this level of protective efficacy in human subjects would be recognized as a major breakthrough. Though it was routinely observed that protection resulted from this experimental irradiated sporozoite vaccine, the sheer power of attenuated sporozoites remained unrecognized until after completion of the careful analysis necessary to publish this report. Interestingly, when these results were presented by one of us (SLH) at the Keystone meeting in March 2002, "Malaria's Challenge: From Infants to Genomics to Vaccines," they were considered interesting, but no one in the audience even raised the idea that this approach should be pursued as viable malaria vaccine, because all thought the vaccine to be impractical to produce and impossible to administer. This view is still widely held in the scientific community. In a recent publication in Nature magazine (October 2, 2003) [49], the director of clinical trials at the Naval Medical Research Center Malaria Program stated, "The barriers have seemed sufficiently daunting that no one has been willing to give it a try," and a malaria vaccine expert from the University of Oxford in the United Kingdom stated, "It's a long shot....It's worth a try, although the odds are heavily stacked against him." In contrast, the inventors believed that it was possible to make such a vaccine, but there were several critical questions that had to be answered before moving into cGMP manufacturing and clinical trials. These are outlined in a recent publication [50] One of the most critical questions was whether one can administer attenuated sporozoites by a route that is practical for a human vaccine?

### SUMMARY OF THE INVENTION

Heretofore, it had been considered impractical to immunize humans with attenuated Plasmodium species sporozoites, because the sporozoites had to be delivered by the bite of infected irradiated mosquitoes for immunization, or by intravenous injection, as this was what had been done previously with humans and mice respectively, and was accepted by the scientific community as the only way to achieve high level protective immunity.

It has been theorized that when properly irradiated sporozoites are delivered by mosquito bite or intravenous injection, they pass through the bloodstream to the liver, invade hepatocytes, partially develop, and then arrest development, never developing to the mature liver schizont, which ruptures, and releases merozoites which cause infection of erythrocytes, and the disease known as malaria. Thus, they are attenuated. Data indicate that in order to elicit adequate protective immune responses, the parasites must invade hepatocytes, partially develop, and express new proteins that are the targets of protective immune responses, particularly CD8 T cells.

The inventors theorized that there is a direct correlation/assocation between the infectivity of a preparation of unirradiated sporozoites and their capacity to elicit protective immunity when they are attenuated. Furthermore, we theorized that there is a direct correlation/assocation between the infectivity of unirradiated sporozoites when administered by a particular method, and the capacity of those sporozoites when irradiated and delivered by that method to elicit protective immunity.

The present invention described herein was discovered in response to asking the question, can one administer the attenuated sporozoites by a route that is practical for a human vaccine?

This question was addressed using the *P. yoelii* rodent malaria parasite, not the *P*. *berghei* rodent malaria parasite, which had been studied previously in all reports cited above (11, 20-22). The *P. berghei* model system was used to establish that irradiated sporozoites protect A/J mice, and this led to the human studies demonstrating that exposure to irradiated *P. falciparum* infected mosquitoes protects humans. The *P. berghei* system was also used to prove to the scientific community that intramuscular, subcutaneous and other non-intravenous routes of administration of irradiated sporozoites are not adequately protective in mice (20-22). In fact after subcutaneous administration of radiation attenuated sporozoites protection was 0% [21]. These studies which were primarily done in A/J mice led to the conclusion that it was not possible to develop irradiated sporozoites as a practical, clinically relevant malaria vaccine for humans. In the early to mid 1980s the Naval Medical Research Institute laboratory switched from working with *P. berghei* in A/J mice to working with *P. yoelii* in BALB/c mice. This was because the scientists at the Naval Medical Research Institute believed that intravenously administered *P. yoelii* in BALB/c mice was more predictive of *P. falciparum* infection in humans than was intravenously administered *P. berghei.* This was in large part because intravenously administered *P. yoelii* sporozoites are so much more infectious to mice than are intravenously administered *P. berghei* sporozoites. The 50% infectious dose to mice of intravenously administered *P. yoelii* in BALB/c mice is approximately 100-1000 times lower than the 50% infectious dose of *P. berghei* in BALB/c mice and almost certainly more comparable to the 50% infectious dose of Plasmodium sp. parasites in primates, such as *P. knowlesi* in monkeys and *P*. *falciparum* in humans than is *P. berghei.* In the early 1990s, approximately 10 years after the Navy group began working with *P. yoelii* instead of P. *berghei,* after reading papers and hearing presentations from scientists from the Navy group, Dr. Nussenzweig requested the *P. yoelii* parasites used by the Navy laboratory from one of the inventors (SLH), and essentially switched the work in her group at New York University on rodent malaria to the *P. yoelii* model system, primarily working with BALB/c mice.

It is important to note that all work with *P. yoelii* has focused on administration by intravenous injection or mosquito bite, almost certainly because of the previous work in the *P*. *berghei* model system described above [11, 20-22]. Furthermore, because of that work in the P. *berghei* model system no one has experimented in the *P. yoelii* system to try to use it as a model to develop an attenuated whole sporozoite vaccine. Immunization with irradiated sporozoites in the *P. yoelii* rodent malaria system has been used by scientists for the same scientific objectives described in 1980 by Nussenzweig [22]; to identify the immune mechanisms of protective immunity and the antigenic targets on the parasite of these protective immune responses. For this reason, since it has been "known" for more than 25 years that only intravenous or mosquito bite administration of sporozoites provides the 100% protective immunity that makes the irradiated sporozoite model so effective, these have been the routes of administration used by scientists working in this system. The other routes (e.g. subcutaneous, intramuscular, intradermal and others) that would be required to make the irradiated sporozoite clinically practical and acceptable have not been used.

The inventors have discovered a method for immunizing subjects against malaria which allows for the vaccination of large numbers of subjects with attenuated sporozoites in a relatively short time, avoids the impracticality and potential danger of the previous methods of bite by infected mosquitoes, or in the case of mice by intravenous injection, and which provides protection comparable to that achieved by these prior methods.

More particularly, we have discovered that effective protection against malaria can be obtained by parentally administering a dosage of attenuated sporozoites to a subject by a route other than intravenous injection, including, but not limited to the subcutaneous, intramuscular, intradermal, mucosal, submucosal, epidermal, and cutaneous routes.

### DETAILED DESCRIPTION OF THE INVENTION

The instant invention provides a new clinically relevant and acceptable method of administering attenuated Plasmodium species sporozoites that makes it practical for attenuated sporozoites to be used as a vaccine to prevent malaria in humans, mammals, avians, and other relevant species.

The invention's significant improvement over previously standard methods of administration, administration by intravenous injection or by the bite of infected mosquitoes, of attenuated sporozoites is that it allows for a clinically practical and safe method of administering the vaccine that provides protection comparable to the previous standard methods. Administration by the bite of infected mosquitoes can never be used as a vaccine for obvious reasons, and administration by intravenous injection is a method that is not in general use for any vaccine, because it is a technically difficult method of administration, especially in young children, and it is potentially dangerous because of direct injection into the bloodstream.

With the present invention, the parenteral administration may be administered in the skin (transcutaneous, epidermally, intradermally), subcutaneous tissue (subcutaneously), muscle (intramuscularly), through the mucous membranes, or in the submucosal tissue Preferably, the administration is subcutaneously, intradermally or intramuscularly.

The goal of attenuation is to weaken the parasites, so that they are viable enough to invade host cells and produce new proteins, but unable to produce a replicating asexual blood stage infection that causes disease. Attenuation can occur in multiple ways. For example this can occur by attenuating the parasites so that inoculated sporozoites can invade host cells, partially develop in these cells, and arrest development before reaching the stage comparable to a mature hepatic stage parasite that can rupture releasing merozoites that invade erythrocytes and cause disease. This type of attenuated parasite can be termed a metabolically active, non-replicating parasite. Attenuation could also occur by producing parasites that can invade and normally develop in host cells to the stage comparable to a mature hepatic stage parasite, rupture from the host cells, but be unable to develop in erythrocytes to the point required for them to cause disease. This could also occur by attenuating the parasites so that they can invade and normally develop in host cells to the stage comparable to a mature hepatic stage parasite, rupture from the host cells, but be unable to develop in erythrocytes to the point required for them to cause significant disease. This could also occur by attenuating the parasites so that sporozoites partially develop and produce new proteins, but arrest development before reaching the stage comparable to a mature hepatic stage parasite that can rupture releasing merozoites that invade erythrocytes and cause disease.

While numerous methods of attenuation may be used, we have found that attenuation by irradiation is currently preferred for producing a metabolically active, non-replicating parasite. Attenuation of the sporozoites can be accomplished in multiple ways with multiple dosage regimens. The attenuation can be accomplished while the sporozoites are still in the mosquito, after they have been isolated from the mosquitoes and before interventions such as cryopreservation, or after they have been isolated from the mosquitoes and after interventions such as cryopreservation. The current dose of irradiation based on previous experience is generally greater than 12,000 Rads (cGy) and less than 23,000 Rads (cGy) for *Plasmodium falciparum* sporozoites with 15,000 Rads (cGy) being most commonly used [34]. One skilled in the art will recognize that this dosage may vary from species to species or strain to strain or with the apparatus and techniques used to irrradiate the sporozoites. One skilled in the art will recognize that the irradiation can be accomplished using numerous methods, including, but not limited to gamma rays, x-rays, ultraviolet rays, or other subatomic particles such as electrons, protons, or combinations of these methods.

In the future, attenuation as described in paragraph [39] above may be achieved by genetic manipulation of the parasites prior to their being introduced into the vaccine recipient.

Attenuation may also be achieved by treating individuals before or after exposure to sporozoites with drugs which prevent development of the parasites so that they can replicate in hepatoctyes.

Attenuation may also be achieved by treating individuals before or after exposure to sporozoites with drugs which prevent development of the parasites so that they can replicate in erythrocytes.

Attenuation may also be achieved by treating the sporozoites with chemicals which attenuate the parasites.

The means of administration may be any methods for inoculation other than by mosquito bite or intravenous administration, such as, but not limited to injection with a single needle and syringe, multiple needles and syringe arrays, micro-needles with one to hundreds to thousands of pores, needleless injection by ballistic techniques, and the like. The attenuated sporozoites may also be delivered by a transcutaneous patch, or on a particulate material, for example, gold beads. While it is possible to achieve a level of protection with a single inoculation, it is preferred that a series of two or more inoculations or exposures be effected.

The preferred inoculant is a malaria immunization effective amount of attenuated *P*. *falciparum* or other Plasmodium species sporozoites. The dosage in humans per inoculation may range from about 1,000 to 10, 000,000, although this may be varied depending on evaluation by the practitioner or the immunogenicity /potency of the attenuated sporozoite preparations.

Any Plasmodium species parasite, even if altered genetically, may be used in the method of the invention. In one embodiment, the parasite is *P. falciparum.* In other embodiments, for example, the parasite may be *P vivax, P. ovale,* or *P. malariae.* In other embodiments it could a mixture of these parasites. In other embodiments it could be *Plasmodiium knowlesi, P. yoelii,* or other Plasmodium species parasites.

In one embodiment the invention provides a pharmaceutical kit comprising the attenuated sporozoites in the delivery instrument such as a syringe.

In other embodiments the invention provides a kit which includes a container such as a vial, but not limited to a vial containing the frozen attenuated sporozoites, a container such as a vial containing fluid to dilute the attenuated sporozoites, and the actual delivery devices, such as a syringe and needle.

In other embodiments the invention provides a kit which includes a container such as a vial, but not limited to a vial containing the freeze-dried (lyophilizied) attenuated sporozoites, a container such as a vial containing fluid to dilute the attenuated sporozoites, and the actual delivery devices, such as a syringe and needle.

In other embodiments the invention provides a kit which includes a container such as a vial, but not limited to a vial containing preserved attenuated sporozoites, a container such as a vial containing fluid to dilute the attenuated sporozoites, and the actual delivery devices, such as a syringe and needle.

The invention further provides the use of parenteral administration of attenuated Plasmodium species sporozoites as described herein, in the administration of a vaccine for prevention or reduction of severity of malaria.

The invention provides partial, enhanced, or full protection of a human who has not previously been exposed to a malaria-causing pathogen, or has been exposed, but is not fully protected. The invention may also be used to reduce the chance of developing a malaria infection, reduce the chance of becoming ill when one is infected, reduce the severity of the illness, such as fever, when one becomes infected, reduce the concentration of parasites in the infected person, or to reduce mortality from malaria when one is exposed to malaria parasites. In many cases even partial protection is beneficial. For example, a vaccine treatment strategy that results in any of these benefits of about 30% of a population may have a significant impact on a the health of a community and of the individuals residing in the community.

A "vaccine" is a composition of matter comprising a preparation that contains an infectious agent or its components which is administered to stimulate an immune response that will protect a person from illness due to that agent. A therapeutic (treatment) vaccine is given after infection and is intended to reduce or arrest disease progression. A preventive (prophylactic) vaccine is intended to prevent initial infection. Agents used in vaccines may be whole-killed (inactive), live-attenuated (weakened) or artificially manufactured. A vaccine may further comprise a diluent, an adjuvant, a carrier, or combinations thereof, as would be readily understood by those in the art.

A vaccine may be comprised of separate components. As used herein, "separate components" refers to a situation wherein the term vaccine actually comprises two discrete vaccines to be administered separately to a subject. In that sense, a vaccine-comprised of separate components may be viewed as a kit or a package comprising separate vaccine components. For example, in the context of the instant invention, a package may comprise an attenuated sporozoite component and recombinant subunit vaccine component, including but not limited to a recombinant protein, recombinant virus, recombinant bacteria, recombinant parasite, DNA vaccine, or RNA vaccine.

An "effective" immunizing dosage may range between 1000 and 10 million sporozoites, but could be lower if the immunogenicity/potency of the vaccine is increased. The vaccine may be administered on multiple occasions. An "effective" number of inoculations may range between 1 and 6 doses within a year, and "booster" doses in subsequent years.

Both the foregoing general description and the following detailed description are exemplary and explanatory only and are not restrictive of the invention, as claimed. Moreover, the invention is not limited to the particular embodiments described, as such may, of course, vary. Further, the terminology used to describe particular embodiments is not intended to be limiting, since the scope of the present invention will be limited only by its claims.

With respect to ranges of values, the invention encompasses each intervening value between the upper and lower limits of the range to at least a tenth of the lower limit's unit, unless the context clearly indicates otherwise. Further, the invention encompasses any other stated intervening values. Moreover, the invention also encompasses ranges excluding either or both of the upper and lower limits of the range, unless specifically excluded from the stated range.

Unless defined otherwise, the meanings of all technical and scientific terms used herein are those commonly understood by one of ordinary skill in the art to which this invention belongs. One of ordinary skill in the art will also appreciate that any methods and materials similar or equivalent to those described herein can also be used to practice or test the invention. Further, all publications mentioned herein are incorporated by reference.

It must be noted that, as used herein and in the appended claims, the singular forms "a," "or," and "the" include plural referents unless the context clearly dictates otherwise. Thus, for example, reference to "an attenuated sporozoite vaccine" includes a plurality of such sporozoites and reference to "the agent" includes reference to one or more agents and equivalents thereof known to those skilled in the art, and so forth.

Further, all numbers expressing quantities of ingredients, reaction conditions, % purity, and so forth, used in the specification and claims, are modified by the term "about," unless otherwise indicated. Accordingly, the numerical parameters set forth in the specification and claims are approximations that may vary depending upon the desired properties of the present invention. At the very least, and not as an attempt to limit the application of the doctrine of equivalents to the scope of the claims, each numerical parameter should at least be construed in light of the number of reported significant digits, applying ordinary rounding techniques. Nonetheless, the numerical values set forth in the specific examples are reported as precisely as possible. Any numerical value, however, inherently contains certain errors from the standard deviation of its experimental measurement.

Obviously, many modifications and variations of the present invention are possible in light of the above teachings. It is therefore to be understood that, within the scope of the appended claims, the invention many be practiced otherwise than as specifically described.

The following examples further illustrate the invention. They are merely illustrative of the invention and disclose various beneficial properties of certain embodiments of the invention. These examples should not be construed as limiting the invention.

### EXAMPLES

### EXAMPLE 1

### Comparative Infectivity of Intradermal, Intramuscular, Subcutaneous and Intravenous Injection of Sporozoites

A study was conducted to investigate the comparative infectivity of freshly dissected sporozoites delivered intradermally (ID), intramuscularly (IM), subcutaneously (SQ) or intravenously (IV). It is noted that IV administration is considered to be the most reliable methods for achieving infection.

Methods: BALB/c mice were infected with *Plasmodium yoelii* sporozoites hand-dissected from salivary glands by ID, IM, SQ, or IV administration. The level of infection was determined by assessing thick blood films from day 1 through day 14 after administration. The results are shown in Table I.

**Table I**

| Group | No. of Spz | No. Mice | No. infected | % infected |
|---|---|---|---|---|
| IV | 100 | 10 | 10 | 100 |
| ID | 100 | 10 | 9 | 90 |
| ID | 500 | 10 | 10 | 100 |
| IM | 500 | 10 | 10 | 100 |
| SQ | 500 | 10 | 10. | 100 |

These data demonstrate that it is possible to routinely infect BALB/c mice by delivery of sporozoites in the skin, muscle, or subcutaneous tissue.

### EXAMPLE 2

### Comparative Infectivity of Multiple Dose of Sporozoites Administered Intradermally, Intramuscularly, Subcutaneously or Intravenously

A study was conducted to investigate the comparative infectivity with lesser numbers of freshly dissected sporozoites than used in Example I.

Methods: BALB/c mice infected with *Plasmodium yoelii* sporozoites hand-dissected from salivary glands by multiple routes [intradermal (ID), intramuscular (IM), subcutaneous (SQ) or intravenous (IV)]. Infection was determined by assessing thick blood films through day 14 after infection. The results are shown in Table II.

**Table II**

| Group | No. of SPZ | No. of mice | No. Infected | % infected |
|---|---|---|---|---|
| IV | 100 | 10 | 10 | 100 |
| | 20 | 10 | 9 | 90 |
| | 4 | 10 | 3 | 30 |
| ID | 100 | 10 | 8 | 80 |
| | 20 | 10 | 3 | 30 |
| | 4 | 10 | 1 | 10 |
| IM | 100 | 10 | 7 | 70 |
| | 20 | 10 | 3 | 30 |
| | 4 | 10 | 1 | 1 |
| SQ | 100 | 10 | 9 | 90 |
| | 20 | 10 | 4 | 40 |
| | 4 | 10 | 0 | 0 |

These data show that administration of small numbers of *Plasmodium yoelii* sporozoites hand-dissected from salivary glands by the ID, IM, or SQ routes leads to infections in mice with nearly the same efficiency as as by the IV route. Since we theorize that there is a direct correlation/assocation between the infectivity of unirradiated sporozoites when administered by a particular method, and the capacity of those sporozoites when irradiated and delivered by that method to to elicit protective immunity, these data suggest that it should be feasible to successfully immunize by the ID, IM, and SQ routes as well as by the standard IV route.

### EXAMPLE 3

### Protective Efficacy of Single Dose of Irradiated Sporozoites Administered by the Intradermal, Intramuscular, or Intraveous Routes

A study was conducted to investigate the comparative protection provided by immunization with a single dose of 150,000 radiation attenuated sporozoites.

Method: BALB/c mice were inoculated with a single dose of 150,000 radiation attenuated (10,000 Rads/cGy) *P. yoelii* sporozoites by the ID, IM, or IV routes. The sporozoites for immunization were obtained by density gradient centrifugation. The inoculated mice were challenged 10 days later by injection of 100 *Plasmodium yoelii* sporozoites hand-dissected from salivary glands. The infections were assessed through day 14 after challenge by thick blood smear. The level of infection was evaluated on a scale of 1+ (barely detectable) to 4+ (heavy infection). The control group received no immunization inoculation. The results are shown in Table III.

**Table III**

| **Group** | **# Mice** | **Day 4 Protected/Challenged** | **Day 4 Level Inf.** | **Day 5 Protected/Challenged** | **Day 5 Level Inf.** | **Day 14 Protected/Challenged** |
|---|---|---|---|---|---|---|
| **Cont** | **8** | **0/8** | **++++** | **0/8** | **++++** | **0/8** |
| **IV** | **6** | **2/6** | **+** | **1/6** | **+** | **0/6** |
| **ID** | **6** | **4/6** | **+** | **2/6** | **+** | **1/6** |
| **IM** | **6** | **3/6** | **+** | **2/6** | **+** | **0/6** |

These data demonstrate that administration of a single dose of irradiated sporozoites by the ID and IM routes elicits a protective immune response that provides protection against sporozoite challenge comparable to the protection seen after administration of a single dose of irradiated sporozoites by the IV route. This finding was predicted by the infectivity demonstrated in Examples 1 and 2 above. Inasmuch as IM and ID methods are more easily used with large numbers of people and the administration can be carried out with much greater safety and ease than by IV administration, the present invention makes possible the effective immunization of significant populations with attenuated sporozoites in a manner more facile than heretofore demonstrated. In fact it makes it possible to conceive of for the first time a practical attenuated sporozoite vaccine. Administration of the single dose of irradiated sporozoites led to a dramatic reduction of parasite burden in the the mice that were challenged, an effect thought by many malaria vaccinologists to potentially be adequate to significantly reduce morbidity and mortality of malaria in recipients. However, it did not completely protect against infection.

### EXAMPLE 4

### Protective Efficacy of Three Doses of Irradiated Sporozoites Administered by the Subcutaneous or Intraveous Routes

A study was conducted to investigate the comparative protection provided by immunization with a standard regimen of three doses of radiation attenuated *Plasmodium yoelii* sporozoites by the ID or IV routes; a regimen expected to elicit complete protection against sporozoite challenge.

Method: BALB/c mice were inoculated with a first dose of 50,000 radiation attenuated (10,000 RADS/cGy) *Plasmodium yoelii* sporozoites by the SQ or IV routes. The mice received two booster doses of 30,000 irradiated sporozoites (total of 110,000 sporozoites divided into 3 doses). The sporozoites for immunization were obtained by density gradient centrifugation. The inoculated mice were challenged 14 days after last booster dose with 100 *Plasmodium yoelii* sporozoites hand-dissected from salivary glands. The infections were assessed through day 14 after challenge by thick blood smear. Infection was assessed as present or absent. The results are shown in Table IV.

**Table IV**

| **Group** | **No. Mice** | **Day 14 Prot/Chal** | **Day 14 % Protected** |
|---|---|---|---|
| **Control** | **8** | **0/8** | **0** |
| **IV** | **7** | **7/7** | **100%** |
| **SQ** | **8** | **8/8** | **100%** |

The data in Table IV clearly demonstrate that one can achieve 100% protection against infection by subcutaneous administration of sporozoites (SQ). These results were predicted by the results of studies shown in Example 1, Example 2, and Example 3, but for the first time ever demonstrated in this experiment. Given the comparability in infectivity by the SQ, ID, and IM routes (Example 2), it seems obvious that administration of sporozoites by those routes would provide comparable protection. The 100% protection reported in Example 4 stands in stark contrast to the 0% protection with subcutaneous immunization of A/J mice with radiation attenuated *P. berghei* sporozoites reported previously [21]. As stated above we believe that our discovery was made possible by our recognition that the *P*. *yoelii*-BALB/c model is more relevant to *P. falciparum* in humans, than is the *P. berghei*-A/J mouse model system.

### EXAMPLE 5

### Infectivity of Sporozoites Isolated by Density Gradient Centrifugation as Compared to by Hand Dissection of Salivary Glands When Administered by the Intravenous Route

In EXAMPLES 3 and 4 the mice were immunized by administration of irradiated sporozoites that had been isolated by density gradient centrifugation. It had been our assumption that sporozoites isolated by density gradient centrifugation of the head and thorax of the mosquitoes are less infective than are sporozoites hand-dissected from salivary glands. If that is the case, and there is a direct association between the infectivity of sporozoites and their capacity to elicit protective immunity as stated above (paragraph [068]), then it should require far fewer sporozoites hand-dissected from salivary glands than sporozoites isolated by density gradient centrifugation to achieve protective immunity. The inventors therefore first conducted an experiment comparing the infectivity of *P. yoelii* sporozoites isolated by density gradient centrifugation to those isolated by hand dissection of salivary glands.

Method: *P. yoelii* sporozoites were isolated from *Anopheles stephensi* mosquitoes by density gradient centrifugation or by hand dissection of salivary glands. BALB/c mice were inoculated by intravenous injection with differing numbers of sporozoites. The infections were assessed through day 14 after challenge by thick blood smear. Infection was assessed as present or absent. The results are shown in Table V.

**Table V**

| **Sporozoites Isolated by Hand Dissection or Density Gradient Centrifugation** | | |
|---|---|---|
| **No. of spz Injected** | **Number Infected/Number Challenged** | |
| | **Hand Dissection** | **Density Gradient Centrifugation** |
| **625** | **10/10** | **7/10** |
| **125** | **10/10** | **4/10** |
| **25** | **10/10** | **0/10** |
| **5** | **5/10** | **0/10** |
| **1** | **0/9** | **0/10** |
| **50% Infectious Dose (ID 50)** | **4.9** | **433** |

The data in Table V clearly demonstrate that sporozoites hand-dissected from salivary glands are more infective than are sporozoites isolated by density gradient centrifugation. The 50% infectious dose is more than 80 times greater for sporozoites isolated by density gradient centrifugation. If the hypothesis is correct that the protective efficacy of a lot of attenuated sporozoites is directly associated with the infectivity of the lot of sporozoites before they were attenuated, then these data would indicate that the numbers of attenuated sporozoites required to achieve protection would be substantially less for sporozoites isolated by hand-dissection of salivary glands sas compared to sporozoites isolated by density gradient centrifugation. which has been the standard way of isolating sporozoites for immunization studies in the P. yoelii-BALB/c model system.

### EXAMPLE 6

### Protective Efficacy of Sporozoites Isolated by Density Gradient Centrifugation as Compared to by Hand Dissection When Administered by the Intravenous Route

Based on the results of the infectivity experiment in EXAMPLE 5, a protective efficacy experiment was designed. The protective efficacy of a regimen of irradiated sporozoites isolated by density gradient centrifugation which was known based on previous-experience to give 90% protection, was compared to the capacity of much lower doses of irradiated sporozoites isolated by hand dissection of salivary glands to achieve protective immunity.

Method: *Anopheles stephensi* mosquitoes infected with *P. yoelii* sporozoites were irradiated with 10,000 Rads/cGy. Sporozoites were isolated by density gradient centrifugation or by hand dissection of salivary glands. BALB/c mice were inoculated by intravenous injection of three doses of irradiated *P. yoelii* sporozoites at 2 week intervals. Group 1 received irradiated sporozoites isolated by density gradient centrifugation (24,000, 8,000, and 8,000 for first, second, and third doses respectively). Groups 2-5 received sporozoites isolated by hand dissection of salivary glands. Group 6 received no immunizations. The mice in Groups 1-6 were challenged with 100 *P. yoelii* sporozoites isolated by hand-dissection of salivary glands 14 days after the third immunizing dose. The infections were assessed through day 14 after challenge by thick blood smear. Infection was assessed as present or absent. The results are shown in Table VI.

**Table VI**

| **Group** | **# Mice** | **# Infected** | **% Protected** |
|---|---|---|---|
| **Density gradient centrifugation 24000 , 8000, 8000 (1)** | **9** | **1** | **88.8%** |
| **Hand-Dissected-18000,6000,6000 (2)** | **10** | **0** | **100%** |
| **Hand-Dissected 9000,3000,3000 (3)** | **10** | **0** | **100%** |
| **Hand-Dissected 4500,4500,4500 (4)** | **10** | **0** | **100%** |
| **Hand-Dissected 4500,1500,1500 (5)** | **10** | **0** | **100%** |
| **Control-Non-immunized mice (6)** | **10** | **10** | **0** |

The data in Table VI demonstrate that mice immunized with a total of 40,000 irradiated *P. yoelii* sporozoites (24000, 8000, 8000) isolated by density gradient centrifugation had 90% protection. Mice immunized with a total of 7500 irradiated sporozoites (4500, 1500, 1500) isolated by hand dissection of salivary glands had 100% protection. These data, when taken with the data in EXAMPLE 5 indicate that there is a direct association between the infectivity of a preparation of sporozoites, and the protective efficacy they can elicit. In fact it is not yet clear how low one can go in terms of doses of irradiated, hand-dissected sporozoites, and still achieve 90%-100% protective efficacy. These data indicate that immunizing with small doses of irradiated sporozoites, whether by the IV, ID, IM, or SQ routes, will lead to protective efficacy.

These data also support the hypothesis that the *P*. *yoelii*-BALB/c model system more closely predicts what occurs in humans with *P. falciparum* than does the *P*. *berghei*-A/J mouse model system, in part because of the much higher infectivity of sporozoites in the *P*. *yoelii* system. Humans can be fully immunized by the bite of 1000 irradiated, *P. falciparum* infected mosquitoes [34]. It is thought that a mosquito inoculates no more than 10 sporozoites when it feeds [51]. If that is the case, then fully immunized and protected humans are probably inoculated with only 10,000 sporozoites [50]. In contrast, in the *P*. *berghei*-A/J mouse model system greater than 100,000 sporozoites isolated from hand-dissected salivary glands were used to achieve protection by intravenous administration, and this immunizing dosage regimen provided no protection when administered subcutaneously [21]. In Example 6 it is demonstrated that administration to BALB/c mice of 7500 *P*. *yoelii* sporozoites isolated by hand dissection of salivary glands provided 100% protection. The fact that BALB/c mice immunized with attenuated *P*. *yoelii* sporozoites and humans immunized with attenuated *P. falciparum* sporozoites are protected after exposure to similar numbers of attenuated sporozoites, and A/J mice immunized with *P*. *berghei* sporozoites are immunized with more than 10 times the quantity of sporozoites, supports our hypothesis that the *P*. *yoelii*-BALB/c model will be more predictive of what will occur in humans than the *P*. *berghei*-A/J model system.

### CONCLUSIONS

The process of developing an effective, sustainable vaccine against infections like *P. falciparum* has proven to be slower, more difficult and complex than expected. There is no licensed malaria vaccine, but it is now known that immunization with radiation attenuated *P*. *falciparum* sporozoites by the bite of greater than a 1000 infected mosquitoes provides sterile protective immunity in greater than 90% of immunized individuals for at least 10.5 months against multiple isolates of *P. falciparum* from throughout the world. One of the major obstacles to making this immunization regimen into a vaccine for humans has been the fact that it is not possible to provide a regulated vaccine to large numbers of individuals by the bite of infected mosquitoes. Furthermore, work by a number of scientists indicated that excellent protection could only be achieved in the mouse model system by intravenous administration of attenuated sporozoites, a method of administration that is not in general used for vaccination, because it is technically difficult and potentially more dangerous than are standard methods of administration. Because methods of administration conventionally used in humans for immunization like subcutaneous and intramuscular inoculation did not lead to adequate protective immunity in this mouse model system, it was heretofore not considered possible to develop an attenuated sporozoite vaccine for humans. Utilizing a different model system than that used by previous investigators, we have discovered a method of administering sporozoites that leads to high level protection and is practical, safe, and accepted. This discovery should facilitate utilization of this method of administering attenuated sporozoites to develop and provide a practical, mass-delivered attenuated sporozoite malaria vaccine.

The following publications as well as those mentioned anywhere else in this application, are hereby cited:
1. Breman JG. Ears of the hippopotamus: manifestations, determinants, and estimates of the malaria burden. Am J Trop Med Hyg 2001; 64:1-11.
2. Gallup JL, Sachs JD. The economic burden of malaria. Am J Trop Med Hyg 2001; 64:85-96.
3. World Tourism Organization. International tourist arrivals by (sub)region. June 2002; http://www.world-tourism.org/market_research/facts&figures/latest_data/tita01_07-02.pdf.
4. Beadle, C. and Hoffman, S. L. History of malaria in the United States Naval Forces at war: World War I through the Vietnam conflict. Clin.Infect.Dis. 16:320-329, 1993.
5. Richie TL, Saul A. Progress and challenges for malaria vaccines. Nature 415(6872):694-701, 2000.
6. Long CA, Hoffman SL. Parasitology: Malaria--from infants to genomics to vaccines. Science 297: 345-7, 2002. '
7. Stoute JA, Kester KE, Krzych U, Wellde BT, Hall T, White K, Glenn G, Ockenhouse CF, Garcon N, Schwenk R, Lanar DE, Sun P, Momin P, Wirtz RA, Golenda C, Slaoui M, Wortmann G, Holland C, Dowler M, Cohen J, Ballou WR. Long-term efficacy and immune responses following immunization with the RTS,S malaria vaccine. J Infect Dis_178: 1139-44, 1998.
8. Kester KE, McKinney DA, Tornieporth N, Ockenhouse CF, Heppner DG, Hall T, Krzych U, Delchambre M, Voss G, Dowler MG, Palensky J, Wittes J, Cohen J, Ballou WR. Efficacy of recombinant circumsporozoite protein vaccine regimens against experimental Plasmodium falciparum malaria. J Infect Dis 183: 640-7, 2001.
9. Bojang KA, Milligan PJ, Pinder M, Vigneron L, Allouche A, Kester KE, Ballou WR, Conway DJ, Reece WH. Efficacy of RTS,S/AS02 malaria vaccine against Plasmodium falciparum infection in semi-immune adult men in The Gambia: a randomised trial. Lancet. 2001 Dec 8;358(9297):1927-34.
10. Dame JB, Williams JL, McCutchan TF, Weber JL, Wirtz RA, Hockmeyer WT, Maloy WL, Haynes JD, Schneider I, Roberts D, Sanders GS, Reddy EP, Diggs CL, Miller LH. Structure of the gene encoding the immunodominant surface antigen on the sporozoite of the human malaria parasite Plasmodium falciparum. Science 225: 593-9, 1984.
11. Nussenzweig RS, Vanderberg J, Most H, Orton C. Protective immunity produced by the injection of X-Irradiated sporozoites of Plasmodium berghei. Nature 216: 160-2, 1967.
12. Clyde DF, Most H, McCarthy VC, Vanderberg JP. Immunization of man against sporozoite-induced falciparum malaria. Am J Med Sci 266: 169-77, 1973.
13. Clyde DF, McCarthy VC, Miller RM, Hornick RB. Specificity of protection of man immunized against sporozoite- induced falciparum malaria. Am J Med Sci 266: 398-401, 1973.
14. Rieckmann KH, Carson PE, Beaudoin RL, Cassells JS, Sell KW. Sporozoite induced immunity in man against an Ethiopian strain of Plasmodium falciparum. Trans R Soc Trop Med Hyg 68: 258-9, 1974.
15. Clyde DF, McCarthy VC, Miller RM, Woodward WE. Immunization of man against falciparum and vivax malaria by use of attenuated sporozoites. Am J Trop Med Hyg 24: 397-401, 1975.
16. McCarthy VC, Clyde DF. Plasmodium vivax: correlation of circumsporozoite precipitation (CSP) reaction with sporozoite-induced protective immunity in man. Exp Parasitol 41: 167-71, 1977.
17. Rieckmann KH, Beaudoin RL, Cassells JS, Sell DW. Use of attenuated sporozoites in the immunization of human volunteers against falciparum malaria. Bull World Health Organ 57: 261-5, 1979.
18. Clyde DF. Immunity to falciparum and vivax malaria induced by Irradiated sporozoites: a review of the University of Maryland studies, 1971-75. Bull World Health Organ 68: 9-12, 1990.
19. Rieckmann KH. Human immunization with attenuated sporozoites. Bull World Health Organ 68: 13-6, 1990.
20. Spitalny GL, Nussenzweig RS. Effect of various routes of immunization and methods of parasite attenuation on the development of protection against sporozoite-induced rodent malaria. Proceedings of the Helminthological Society of Washington. 39 (Special Issue): 506-514, 1972.
21. Kramer LD, Vanderberg JP. Intramuscular immunization of mice with irradiated Plasmodium berghei sporozoites: Enhancement of protection with albumin. Am J Trop Med Hyg. 24 (6): 913-916, 1975.
22. Nussenzweig R. Use of radiation-attenuated sporozoites in the immunoprophylaxis of malaria. International Journal of Nuclear Medicine and Biology 7:89-96, 1980.
23. Trager W, Jensen JB. Culture of human malaria parasites Plasmodium falciparum. Science 193(4254): 673-5, 1976.
24. Haynes JD, Diggs CL, Hines FA, Desjardins RE. Human malaria parasites in continuous culture. Nature 263(5580):767-9, 1976.
25. Campbell CC, Collins WE, Nguyen-Dinh P, Barber A, Broderson JR. Plasmodium falciparum gametocytes from culture in vitro develop to sporozoites that are infectious to primates. Science 217(4564):1048-50, 1982.
26. Chulay JD, Schneider I, Cosgriff TM, Hoffman SL, Ballou WR, Ouakyi IA, Carter R, Trosper JH, Hockmeyer WT. Malaria transmitted to humans by mosquitoes infected from cultured Plasmodium falciparum. Am J Trop Med Hyg. 1986 Jan;35(1):66-8.
27. Yoshida N, Nussenzweig RS, Potocnjak P, Nussenzweig V, Aikawa M. Hybridoma produces protective antibodies directed against the sporozoite stage of malaria parasite. Science 207(4426):71-3, 1980
28. Hilleman MR. Yeast recombinant hepatitis B vaccine. Infection 15(1):3-7, 1987.
29. Ballou WR, Hoffman SL, Sherwood JA, Hollingdale MR, Neva FA, Hockmeyer WT, Gordon DM. Safety and efficacy of a recombinant DNA Plasmodium falciparum sporozoite vaccine. Lancet 1(8545):1277-81, 1987.
30. Herrington DA, Clyde DF, Losonsky G, Cortesia M, Murphy JR, Davis J, Bager S, Felix AM. Safety and immunogenicity in man of a synthetic peptide malaria vaccine against Plasmodium falciparum sporozoites. Nature 328(6127):257-9, 1987.
31. Nussenzweig V, Nussenzweig RS. Rationale for the development of an engineered sporozoite malaria vaccine. Adv Immunol 45: 283-334, 1989.
32. Hoffman SL, Franke ED, Hollingdale MR, Druilhe P. Attacking the infected hepatocyte. In: Hoffman SL, ed. Malaria Vaccine Development: A Multi-Immune Response Approach. Washington, D.C.: ASM Press, pp. 35-75, 1996.
33. Hoffman SL, Miller LH. Perspectives on malaria vaccine development. In: Hoffman SL, ed. Malaria Vaccine Development: a Multi-Immune Response Approach. Washington, D.C.: ASM Press, pp. 1-13, 1996.
34. Hoffman SL, Goh LM, Luke TC, Schneider I, Le TP, Doolan DL, Sacci J, de la Vega P, Dowler M, Paul C, Gordon DM, Stoute JA, Church LW, Sedegah M, Heppner DG, Ballou WR, Richie TL. Protection of humans against malaria by immunization with radiation-attenuated Plasmodium falciparum sporozoites. J Infect Dis 185: 1155-64, 2002.
35. Egan JE, Hoffman SL, Haynes JD, Sadoff JC, Schneider I, Grau GE, Hollingdale MR, Ballou WR, Gordon DM. Humoral immune responses in volunteers immunized with Irradiated Plasmodium falciparum sporozoites. Am J Trop Med Hyg 49: 166-73, 1993.
36. Malik A, Egan JE, Houghten RA, Sadoff JC, Hoffman SL. Human cytotoxic T lymphocytes against the Plasmodium falciparum circumsporozoite protein. Proc Natl Acad Sci USA 88: 3300-4, 1991.
37. Wizel B, Houghten RA, Parker K, Coligan JE, Church P, Gordon DM, Ballou WR, Hoffman SL. Irradiated sporozoite vaccine induces HLA-B8-restricted cytotoxic T lymphocyte responses against two overlapping epitopes of the Plasmodium falciparum surface sporozoite protein 2. J Exp Med 182: 1435-45, 1995.
38. Wizel B, Houghten R, Church P, Tine JA, Lanar DE, Gordon DM, Ballou WR, Sette A, Hoffman SL. HLA-A2-restricted cytotoxic T lymphocyte responses to multiple Plasmodium falciparum sporozoite surface protein 2 epitopes in sporozoite-immunized volunteers. J Immunol 155: 766-75, 1995.
39. Krzych U, Lyon JA, Jareed T, Schneider I, Hollingdale MR, Gordon DM, Ballou WR. T lymphocytes from volunteers immunized with Irradiated Plasmodium falciparum sporozoites recognize liver and blood stage malaria antigens. J Immuno/ 155: 4072-7, 1995.
40. Doolan DL, Hoffman SL, Southwood S, Wentworth PA, Sidney J, Chestnut RW, Keogh E, Apella E, Nutman TB, Lal AA, Gordon DM, Oloo A, Sette A. Degenerate cytotoxic T cell epitopes from P. falciparum restricted by HLA-A and HLA-B supertypes alleles. Immunity 7: 97-112, 1997.
41. Doolan DL, Southwood S, Chesnut R, Appella E, Gomez E, Richards A, Higashimoto YI, Maewal A, Sidney J, Gramzinski RA, Mason C, Koech D, Hoffman SL, Sette A. HLA-DR-promiscuous T cell epitopes from Plasmodium falciparum pre- erythrocytic-stage antigens restricted by multiple HLA class II alleles. J Immunol 165: 1123-37, 2000.
42. Herrington D, Davis J, Nardin E, Beier M, Cortese J, Eddy H, Losonsky G, Hollingdale M, Sztein M, Levine M, Nussenzweig RS, Clyde D, Edelman R. Successful immunization of humans with Irradiated sporozoites: humoral and cellular responses of the protected individuals. Am J Trop Med Hyg 45: 539-47, 1991.
43. Edelman R, Hoffman SL, Davis JR, Beier M, Sztein MB, Losonsky G, Herrington DA, Eddy HA, Hollingdale MR, Gordon DM, Clyde DF. Long-term persistence of sterile immunity in a volunteer immunized with X-Irradiated Plasmodium falciparum sporozoites. J Infect Dis 168: 1066-70, 1993.
44. Nardin EH, Herrington DA, Davis J, Levine M, Stuber D, Takacs B, Caspers P, Barr P, Altszuler R, Clavijo P, Nussenzweig RS. Conserved repetitive epitope recognized by CD4+ clones from a malaria-immunized volunteer. Science 246: 1603-6, 1989.
45. Nardin EH. T cell responses in a sporozoite-immunized human volunteer and a chimpanzee. Immunol Lett 25: 43-8, 1990.
46. Nardin EH, Nussenzweig RS, Altszuler R, Herrington D, Levine M, Murphy J, Davis J, Bathurst I, Barr P, Romero P, Zavala F. Cellular and humoral immune responses to a recombinant P. falciparum CS protein in sporozoite-immunized rodents and human volunteers. Bull World Health Organ 68: 85-7, 1990.
47. Moreno A, Clavijo P, Edelman R, Davis J, Sztein M, Herrington D, Nardin E. Cytotoxic CD4+ T cells from a sporozoite-immunized volunteer recognize the Plasmodium falciparum CS protein. Int Immunol 3: 997-1003, 1991.
48. Moreno A, Clavijo P, Edelman R, Davis J, Sztein M, Sinigaglia F, Nardin E. CD4+ T cell clones obtained from Plasmodium falciparum sporozoite-immunized volunteers recognize polymorphic sequences of the circumsporozoite protein. J Immunol 151: 489-99 1993.
49. Butler D. Mosquito production mooted as fast track to malaria vaccine. Nature 435-437,2003.
50. Luke TC, Hoffman SL. Rationale and Plans for Developing a Non-Replicating, Metabolically Active Radiation Attenuated Plasmodium falciparum Sporozoite Vaccine. Journal of Experimental Biology 206:3803-3808, 2003.
51. Beier, JC., et al. Quantitation of Plasmodium falciparum sporozoites transmitted in vitro by experimentally infected Anopheles gambiae and Anopheles stephensi. Am J Trop Med Hyg 44(5): 564-70, 1991.

## Claims

1. A vaccine comprising isolated, metabolically active, attenuated *Plasmodium* sporozoite parasite species, said species selected from the group consisting of *Plasmodium falciparum* and *plasmodium vivax,* and a carrier for use in stimulating an immune response and conferring protective immunity in human hosts by parenteral, non-intravenous administration.

2. The vaccine of claim 1, wherein said sporozoite parasites are obtained from hand-dissected *Anopheles* mosquito salivary glands.

3. The vaccine of any one of claims 1 to 2, comprising *Plasmodium falciparum* sporozoites and at least one additional species of *Plasmodium* sporozoite.

4. The vaccine of any one of claims 1 to 3, wherein said sporozoite parasites are capable of invading cells of said hosts.

5. The vaccine of claim 4, wherein said cells comprise hepatic cells, said sporozoite parasites induce hepatic cell rupture, and said sporozoite parasites are not capable of subsequent development within host erythrocytes.

6. The vaccine of any one of claims 1 to 5, wherein attenuation is achieved by a means of gene alteration.

7. The vaccine of claim 6, wherein said means is selected from the group consisting of irradiation, genetic manipulation, and treatment of sporozoites with chemicals.

8. The vaccine of any one of claims 1 to 7, comprising radiation-attenuated *Plasmodium* sporozoites.

9. The vaccine of claim 8, wherein dosage of attenuating radiation is at least 12,000 cGy and no more than 23,000 cGy.

10. The vaccine of claim 9, wherein the dosage of attenuating radiation is proximate to 15,000 cGy.

11. The vaccine of any one of claims 1 to 10, comprising at least 1000 sporozoites, but not more than 10,000,000, sporozoites.

12. The vaccine of any one of claims 1 to 11, useful for administration to human hosts to prevent malaria-specific pathology in said hosts after subsequent introduction into said hosts of infectious *Plasmodium* sporozoites.

13. The vaccine of claim 1 wherein said protective immunity is sufficient to prevent malaria in substantially all of said hosts after subsequent challenge with a *Plasmodium* parasite.

14. A pharmaceutical vaccination kit for stimulating an immune response and conferring protective immunity in human hosts against one or more malaria-causing pathogens, said kit comprising a vaccine of isolated, metabolically active, attenuated *Plasmodium* sporozoite parasites according to claims 1 to 13, a carrier, and means for parenteral, non-intravenous administration.

15. The vaccination kit of claim 14, wherein said means is a needle.

16. The vaccination kit of claim 14, wherein said means is a micro-needle array.

17. The vaccination kit of claim 14, wherein said means is a needle-free ballistic injector.

18. The vaccination kit of any one of claims 14 to 17, wherein said vaccine is useful for administration to human hosts, to prevent malaria-specific pathology in said hosts, after subsequent introduction into said hosts of infectious *Plasmodium* sporozoites.

19. The vaccination kit of claim 14, wherein said protective immunity is sufficient to prevent malaria in substantially all of said hosts after subsequent challenge with a *Plasmodium* parasite.

20. A composition comprising isolated metabolically active attenuated Plasmodium sporozoite parasites according to claims 1 to 13 for use in eliciting an immune response and conferring protective immunity in human hosts against one or more malaria-causing pathogens, by parenteral, non-intravenous administration of at least one vaccine dose.

21. The composition of claim 20, formulated for subsequent administration to said hosts of one or more vaccine booster doses.

22. The composition of claim 20 or claim 21, wherein the vaccine dose comprises a *Plasmodium-specific* subunit component selected from the group consisting of native protein, recombinant protein, recombinant virus, recombinant bacteria, recombinant parasite, DNA and RNA.

23. The composition of any one of claims 20 to 22, wherein said immune response is therapeutic for a host infected with *Plasmodium* species sporozoites.

24. The composition of any one of claims 20 to 23, wherein said composition is formulated to mitigate malaria-specific pathology in said hosts, said pathology resulting from introduction into said hosts of infectious *Plasmodium* sporozoites subsequent to said administration of said vaccine dose.

25. The composition of any one of claims 20 to 23, wherein said composition prevents malaria-specific pathology in said host, after introduction into said hosts of infectious *Plasmodium* sporozoites subsequent to said administration of said vaccine dose.

26. The composition of any one of claims 20 to 25, wherein said administration is a host-tissue inoculation chosen from a group consisting of subcutaneous, dermal, muscular, epidermal, mucosal, submucosal, and cutaneous administration.

27. The composition of any one of claims 20 to 26, wherein said sporozoite parasites are further isolated from a single species selected from the group consisting of *Plasmodium falciparum, Plasmodium vivax, Plasmodium ovale, Plasmodium knowlesi* and *Plasmodium malariae.*

28. The composition of any one of claims 20 to 26, wherein said sporozoite parasites are further isolated from at least two species selected from the group consisting of *Plasmodium falciparum, Plasmodium vivax, Plasmodium ovale, Plasmodium knowlesi* and *Plasmodium malariae.*

29. The composition of claim 20, wherein said sporozoites are irradiated while within mosquitoes.

30. The composition of claim 21, wherein said one or more vaccine booster doses comprise at least 1000 sporozoites, but no more than 10,000,000, sporozoites.

## Patentansprüche

1. Impfstoff mit isolierten, metabolisch aktiven, attenuierten parasitären Spezies von *Plasmodium-Sporozoiten,* wobei die Spezies aus der aus *Plasmodium falciparum* und *Plasmodium* vivax bestehenden Gruppe ausgewählt sind, und mit einem Träger, zur Anwendung bei der Stimulierung einer Immunantwort und zum Verleihen von schützender Immunität in menschlichen Wirten durch parenterale, nicht-intravenöse Verabreichung.

2. Impfstoff nach Anspruch 1, wobei die Sporozoiten-Parasiten aus manuell sezierten Speicheldrüsen der *Anopheles-Mücke* gewonnen werden.

3. Impfstoff nach einem der Ansprüche 1 bis 2, mit *Plasmodium falciparum-*Sporozoiten und mindestens einer zusätzlichen Spezies von *Plasmodium-Sporozoiten.*

4. Impfstoff nach einem der Ansprüche 1 bis 3, wobei die Sporozoiten-Parasiten in der Lage sind, in Zellen anderer Wirte einzudringen.

5. Impfstoff nach Anspruch 4, wobei die Zellen hepatische Zellen umfassen, die Sporozoiten-Parasiten ein Platzen von Leberzellen herbeiführen, und die Sporozoiten-Parasiten zu einer anschließenden Entwicklung innerhalb von Wirts-Erythrozyten nicht fähig sind.

6. Impfstoff nach einem der Ansprüche 1 bis 5, wobei eine Attenuierung durch ein Mittel zur Genveränderung erzielt wird.

7. Impfstoff nach Anspruch 6, wobei das Mittel aus der Gruppe, die aus Bestrahlung, Genmanipulation und Behandlung von Sporozoiten mit Chemikalien besteht, ausgewählt ist.

8. Impfstoff nach einem der Ansprüche 1 bis 7, mit durch Bestrahlung attenuierten *Plasmodium Sporozoiten.*

9. Impfstoff nach Anspruch 8, wobei eine Dosierung einer Attenuierungsbestrahlung mindestens 12.000 cGy und nicht mehr als 23.000 cGy beträgt.

10. Impfstoff nach Anspruch 9, wobei die Dosierung der Attenuierungsbestrahlung annähernd 15.000 cGy beträgt.

11. Impfstoff nach einem der Ansprüche 1 bis 10, mit mindestens 1.000 Sporozoiten, aber nicht mehr als 10.000.000 Sporozoiten.

12. Impfstoff nach einem der Ansprüche 1 bis 11, nützlich zur Verabreichung an menschliche Wirte, um eine malariaspezifische Pathologie in den Wirten im Gefolge eines Eindringens infektiöser Plasmodium-Sporozoiten in die Wirte zu verhindern.

13. Impfstoff nach Anspruch 1, wobei die schützende Immunität ausreichend ist, um im Wesentlichen bei allen Wirten Malaria im Gefolge einer Konfrontation mit einem *Plasmodium*-Parasiten zu verhindern.

14. Pharmazeutische Impfausrüstung zum Stimulieren einer Immunantwort und zum Verleihen schützender Immunität in menschlichen Wirten gegen einen oder mehrere Malaria verursachende(n) Krankheitserreger, wobei die Ausrüstung umfasst: einen Impfstoff aus isolierten, metabolisch aktiven attenuierten *Plasmodium-*Sporozoiten-Parasiten gemäß den Ansprüchen 1 bis 13, einen Träger und ein Mittel zur parenteralen, nicht-intravenösen Verabreichung.

15. Impfausrüstung nach Anspruch 14, wobei das Mittel eine Nadel ist.

16. Impfausrüstung nach Anspruch 14, wobei das Mittel eine Mikronadel-Anordnung ist.

17. Impfausrüstung nach Anspruch 14, wobei das Mittel ein nadelfreier ballistischer Injektor ist.

18. Impfausrüstung nach einem der Ansprüche 14 bis 17, wobei der Impfstoff nützlich ist zur Verabreichung an menschliche Wirte, um eine malariaspezifische Pathologie in den Wirten im Gefolge eines Eindringens infektiöser *Plasmodium-*Sporozoiten in die Wirte zu verhindern.

19. Impfausrüstung nach Anspruch 14, wobei die schützende Immunität ausreichend ist, um im Wesentlichen bei allen Wirten Malaria im Gefolge einer Konfrontation mit einem *Plasmodium*-Parasiten zu verhindern.

20. Zusammensetzung mit isolierten, metabolisch aktiven, attenuierten *Plasmodium*-Sporozoiten-Parasiten gemäß den Ansprüchen 1 bis 13 zur Anwendung beim Hervorrufen einer Immunantwort und zum Verleihen schützender Immunität bei menschlichen Wirten gegen einen oder mehrere Malaria auslösende(n) Krankheitserreger durch parenterale, nicht-intravenöse Verabreichung mindestens einer Impfstoff-Dosis.

21. Zusammensetzung nach Anspruch 20, formuliert für eine anschließende Verabreichung einer oder mehrerer Impfstoff-Auffrischungsdosen an die Wirte.

22. Zusammensetzung nach Anspruch 20 oder Anspruch 21, wobei die Impfstoffdosis eine Plasmodium-spezifische Untereinheits-Komponente umfasst, die aus der aus nativem Protein, rekombinantem Protein, rekombinanten Viren, rekombinanten Bakterien, rekombinanten Parasiten, DNA und RNA bestehenden Gruppe ausgewählt ist.

23. Zusammensetzung nach einem der Ansprüche 20 bis 22, wobei die Immunantwort für einen mit Sporozoiten der *Plasmodium*-Spezies infizierten Wirt therapeutisch ist.

24. Zusammensetzung nach einem der Ansprüche 20 bis 23, wobei die Zusammensetzung so formuliert ist, dass sie eine malariaspezifische Pathologie in den Wirten mildert, wobei sich die Pathologie aus dem Eindringen von infektiösen Plasmodium-Sporozoiten nach der Verabreichung der Impfstoffdosis ergibt.

25. Zusammensetzung nach einem der Ansprüche 20 bis 23, wobei die Zusammensetzung eine malariaspezifische Pathologie im Wirt nach dem Eindringen von infektiösen *Plasmodium*-Sporozoiten in die Wirte nach der Verabreichung der Impfstoffdosis verhindert.

26. Zusammensetzung nach einem der Ansprüche 20 bis 25, wobei die Verabreichung ein Einspritzen in ein Wirtsgewebe, ausgewählt aus einer Gruppe bestehend aus subkutaner, dermaler, muskulärer, epidermaler, mukosaler, submukosaler und kutaner Verabreichung, ist.

27. Zusammensetzung nach einem der Ansprüche 20 bis 26, wobei die Sporozoiten-Parasiten ferner aus einer einzelnen Spezies, ausgewählt aus der aus *Plasmodium falciparum, Plasmodium vivax, Plasmodium ovale, Plasmodium knowlesi* und *Plasmodium malariae* bestehenden Gruppe, isoliert werden.

28. Zusammensetzung nach einem der Ansprüche 20 bis 26, wobei die Sporozoiten-Parasiten ferner aus mindestens zwei Spezies, ausgewählt aus der aus *Plasmodium falciparum, Plasmodium vivax, Plasmodium ovale, Plasmodium knowlesi* und *Plasmodium malariae* bestehenden Gruppe, isoliert werden.

29. Zusammensetzung nach Anspruch 20, wobei die Sporozoiten bestrahlt werden, während sie sich in Moskitos befinden.

30. Zusammensetzung nach Anspruch 21, wobei die eine oder die mehreren Impfstoff-Auffrischungsdosen mindestens 1000 Sporozoiten, aber nicht mehr als 10.000.000 Sporozoiten umfassen.

## Revendications

1. Vaccin comprenant une espèce isolée, métaboliquement active, atténuée, du parasite sporozoïtaire *Plasmodium,* ladite espèce choisie parmi le groupe consistant en *Plasmodium falciparum* et *Plasmodium vivax,* et un support, destiné à une utilisation pour stimuler une réponse immunitaire et conférer une immunité protectrice chez des hôtes humains par administration parentérale, non intraveineuse.

2. Vaccin selon la revendication 1, dans lequel lesdits parasites sporozoïtaires sont obtenus à partir de glandes salivaires disséquées à la main du moustique *Anopheles.*

3. Vaccin selon l'une quelconque des revendications 1 à 2, comprenant des sporozoïtes de *Plasmodium falciparum* et au moins une espèce additionnelle de sporozoïtes de *Plasmodium.*

4. Vaccin selon l'une quelconque des revendications 1 à 3, dans lequel lesdits parasites sporozoïtaires sont aptes à envahir des cellules desdits hôtes.

5. Vaccin selon la revendication 4, dans lequel lesdites cellules comprennent des cellules hépatiques, lesdits parasites sporozoïtaires induisent une rupture des cellules hépatiques, et lesdits parasites sporozoïtaires ne sont pas aptes à un développement subséquent dans les érythrocytes de l'hôte.

6. Vaccin selon l'une quelconque des revendications 1 à 5, dans lequel l'atténuation est réalisée par un moyen d'altération génique.

7. Vaccin selon la revendication 6, dans lequel ledit moyen est choisi parmi le groupe consistant en une irradiation, une manipulation génétique, et un traitement des sporozoïtes avec des produits chimiques.

8. Vaccin selon l'une quelconque des revendications 1 à 7, comprenant des sporozoïtes de *Plasmodium* atténués par radiation.

9. Vaccin selon la revendication 8, dans lequel le dosage de la radiation d'atténuation est au moins 12 000 cGy et pas plus de 23 000 cGy.

10. Vaccin selon la revendication 9, dans lequel le dosage de la radiation d'atténuation est proche de 15 000 cGy.

11. Vaccin selon l'une quelconque des revendications 1 à 10, comprenant au moins 1 000 sporozoïtes, mais pas plus de 10 000 000 de sporozoïtes.

12. Vaccin selon l'une quelconque des revendications 1 à 11, utile pour une administration à des hôtes humains pour prévenir une pathologie spécifique du paludisme chez lesdits hôtes après introduction subséquente dans lesdits hôtes de sporozoïtes infectieux de *Plasmodium.*

13. Vaccin selon la revendication 1 dans lequel ladite immunité protectrice est suffisante pour prévenir le paludisme chez sensiblement la totalité desdits hôtes après un test de provocation subséquent avec un parasite *Plasmodium.*

14. Trousse pharmaceutique de vaccination pour stimuler une réponse immunitaire et conférer une immunité protectrice chez des hôtes humains contre un ou plusieurs pathogènes causes du paludisme, ladite trousse comprenant un vaccin de parasites sporozoïtaires de *Plasmodium* isolés, métaboliquement actifs, atténués, selon les revendications 1 à 13, un support, et un moyen pour une administration parentérale, non intraveineuse.

15. Trousse de vaccination selon la revendication 14, dans laquelle ledit moyen est une aiguille.

16. Trousse de vaccination selon la revendication 14, dans laquelle ledit moyen est une matrice de micro-aiguilles.

17. Trousse de vaccination selon la revendication 14, dans laquelle ledit moyen est un injecteur balistique sans aiguille.

18. Trousse de vaccination selon l'une quelconque des revendications 14 à 17, dans laquelle ledit vaccin est utile pour une administration à des hôtes humains, pour prévenir une pathologie spécifique du paludisme chez lesdits hôtes, après introduction subséquente dans lesdits hôtes de sporozoïtes infectieux de *Plasmodium.*

19. Trousse de vaccination selon la revendication 14, dans laquelle ladite immunité protectrice est suffisante pour prévenir le paludisme chez sensiblement la totalité desdits hôtes après un test de provocation subséquent avec un parasite *Plasmodium.*

20. Composition comprenant des parasites sporozoïtaires de *Plasmodium* isolés, métaboliquement actifs, atténués, selon les revendications 1 à 13 destinée à une utilisation pour stimuler une réponse immunitaire et conférer une immunité protectrice chez des hôtes humains contre un ou plusieurs pathogènes causes du paludisme, par administration parentérale, non intraveineuse d'au moins une dose de vaccin.

21. Composition selon la revendication 20, formulée pour une administration subséquente auxdits hôtes d'une ou plusieurs doses de rappel de vaccin.

22. Composition selon la revendication 20 ou 21, dans laquelle la dose de vaccin comprend un composant de sous-unité spécifique à *Plasmodium* choisi parmi le groupe consistant en une protéine native, une protéine recombinante, un virus recombinant, une bactérie recombinante, un parasite recombinant, un ADN et un ARN.

23. Composition selon l'une quelconque des revendications 20 à 22, dans laquelle ladite réponse immunitaire est thérapeutique pour un hôte infecté avec des sporozoïte de l'espèce *Plasmodium.*

24. Composition selon l'une quelconque des revendications 20 à 23, dans laquelle ladite composition est formulée pour limiter une pathologie spécifique au paludisme chez lesdits hôtes, ladite pathologie résultant de l'introduction dans lesdits hôtes de sporozoïtes infectieux de *Plasmodium* suite à ladite administration de ladite dose de vaccin.

25. Composition selon l'une quelconque des revendications 20 à 23, dans laquelle ladite composition prévient une pathologie spécifique au paludisme chez ledit hôte, après l'introduction dans lesdits hôtes de sporozoïtes infectieux de *Plasmodium* suite à ladite administration de ladite dose de vaccin.

26. Composition selon l'une quelconque des revendications 20 à 25, dans laquelle ladite administration est une inoculation du tissu hôte choisie parmi un groupe consistant en une administration par voie sous-cutanée, dermique, musculaire, épidermique, muqueuse, sous-muqueuse et cutanée.

27. Composition selon l'une quelconque des revendications 20 à 26, dans laquelle lesdits parasites sporozoïtaires sont en outre isolés à partir d'une espèce unique choisie parmi le groupe consistant en *Plasmodium falciparum, Plasmodium vivax, Plasmodium ovale, Plasmodium knowlesi* et *Plasmodium malariae.*

28. Composition selon l'une quelconque des revendications 20 à 26, dans laquelle lesdits parasites sporozoïtaires sont en outre isolés à partir d'au moins deux espèces choisies parmi le groupe consistant en *Plasmodium falciparum, Plasmodium vivax, Plasmodium ovale,* Plas*modium knowlesi et Plasmodium malariae.*

29. Composition selon la revendication 20, dans laquelle lesdits sporozoïtes sont irradiés alors qu'ils sont à l'intérieur des moustiques.

30. Composition selon la revendication 21, dans laquelle ladite une ou lesdites plusieurs doses de rappel de vaccin comprennent au moins 1 000 sporozoïtes, mais pas plus de 10 000 000 de sporozoïtes.
